# EUROPÄISCHE PATENTSCHRIFT

(11) **EP 0 883 369 B1**
(45) Veröffentlichungstag und Bekanntmachung des Hinweises auf die Patenterteilung: **19.01.2000**
(21) Anmeldenummer: 97906131.4
(22) Anmeldetag: 26.02.1997
(51) Int. Cl.: A47L 13/16

(54) **REINIGUNGSVORRICHTUNG ZUM REINIGEN VON FLÄCHIGEN GEGENSTÄNDEN**
DEVICE FOR CLEANING FLAT OBJECTS
DISPOSITIF POUR LE NETTOYAGE D'OBJETS PLATS

(30) Priorität: 26.02.1996 DE 19607209
(43) Veröffentlichungstag der Anmeldung: 16.12.1998
(73) Patentinhaber: KOHLRUSS, Gregor, D-46325 Borken (DE); WIESNER, Hubert, D-46354 Südlohn (DE); Griebe, Oliver, 46414 Rhede (DE)
(72) Erfinder: KOHLRUSS, Gregor, D-46325 Borken (DE); WIESNER, Hubert, D-46354 Südlohn (DE); LERSCH, Ulrich, D-50259 Pulheim (DE); GRIEBE, Oliver, D-46414 Rhede (DE)
(74) Vertreter: Schneiders, Josef, Dipl.-Ing.
(86) Internationale Anmeldenummer: EP9700920
(87) Internationale Veröffentlichungsnummer: WO9730624

(56) Entgegenhaltungen:
- WO-A-90/15567
- DE-A- 2 925 658
- DE-A- 4 330 357
- US-A- 4 821 360
- US-A- 5 154 524
- US-A- 5 213 430

## Beschreibung

Die vorliegende Erfindung betrifft eine Reinigungsvorrichtung zum Reinigen von flächigen Gegenständen, insbesondere steriler Bereiche in Krankenhäusern, Labors und Arztpraxen, wobei die Reinigungsvorrichtung einen mit mindestens einer ebenen Reinigungsfläche und mindestens einer Auslaß- und Einfüllöffnung versehenen und mit Reinigungs- oder Desinfektionsmittel gefüllten Hohlkörper aufweist, an dem saugfähiges Reinigungsmaterial in Form eines Reinigungskissens befestigt ist, welches in einem Stück von dem Hohlkörper abtrennbar ist.

Derartige Reinigungsvorrichtungen (vgl. z.B. WO-A-90 15 567) haben den Vorteil, daß sie einfach zu handhaben sind und einen hohen Reinigungseffekt haben. Zum Reinigen wird das Reinigungs- oder Desinfektionsmittel auf die zu reinigenden flächigen Gegenstände aufgetragen. Anhaftender Schmutz oder angetrocknete Schmutzreste lassen sich dann mit Hilfe des die Flüssigkeit aufsaugenden, verhältnismäßig dicken Reinigungskissen schnell und einfach entfernen. Ein Nachteil dieser vorbekannten Reinigungsvorrichtung besteht allerdings darin, daß das Reinigungskissen für die Reinigungsarbeit nicht ausreichend fest an dem Hohlkörper festgelegt ist. Das Reinigungskissen ist nämlich lediglich in eine Vertiefung an der Oberfläche des Hohlkörpers eingesetzt, so daß die Gefahr besteht, daß das Reinigungskissen sich unter Einwirkung der bei der Reinigungsarbeit auftretenden Scherkräfte verformt und seitlich aus der Vertiefung herausgedrückt wird.

Aus der DE-A-29 25 658 ist eine ähnliche Reinigungsvorrichtung für flächige Gegenstände bekannt. Sie weist einen mit Reinigungsmittel gefüllten quaderförmigen Hohlkörper mit einer Auslaß- und Einfüllöffnung auf, der an zwei sich gegenüberliegenden Reinigungsflächen mit einem Reinigungsmaterial versehen ist. Das Reinigungsmaterial besteht hier aus einem Papierblock, dessen Blätter nach dem Reinigen eines Gegenstandes einzeln von dem Papierblock abtrennbar sind, um stets eine saubere Oberfläche des Reinigungsmaterials zu gewährleisten. Das Reinigungsmaterial ist saugfähig, um möglichst viel Reinigungswasser bzw. Reinigungsmittel aufnehmen zu können, dieses während des Reinigungsvorganges nach und nach an die zu reinigenden flächigen Gegenstände abzugeben und um dann nach dem Reinigen möglichst viel Schmutzwasser von den gereinigten flächigen Gegenständen aufzunehmen.

Die Saugfähigkeit des Reinigungsmaterials beruht auf der Kapillarwirkung in den Gewebeporen des Reinigungsmaterials. Während des Reinigungsvorganges werden von dem saugfähigen Reinigungsmaterial mit dem Schmutzwasser auch Keime von den zu reinigenden Gegenständen aufgenommen und in den Poren gespeichert. Durch das Entfernen des obersten Blattes des Papierblockes kann zwar die Oberfläche des Reinigungsmaterials sauber gehalten werden. Die in die tiefer liegenden Schichten des Reinigungsmaterials eingedrungenen Keime können indessen nicht aus den Poren des Reinigungsmaterials entfernt werden. Trotz einer scheinbar sauberen Oberfläche des Reinigungsmaterials werden bei der bekannten Reinigungsvorrichtung die Keime von einem zu reinigenden Gegenstand auf den nächsten übertragen.

Dies ist insbesondere in sterilen Bereichen in Krankenhäusern, Labors und Arztpraxen ein großes Problem. Beim Reinigen von Gegenständen in diesen Bereichen ist die Keimfreiheit oberstes Gebot. Es muß verhindert werden, daß Krankheitserreger von einem Krankenzimmer in ein anderes verschleppt werden. Dies ließe sich idealerweise dadurch realisieren, daß auf die Saugfähigkeit des Reinigungsmaterials ganz verzichtet wird. Dies würde den Reinigungseffekt der Reinigungsvorrichtung jedoch erheblich herabsetzen. Schließlich ist es durchaus erwünscht, daß das Reinigungsmaterial nach dem Reinigen eines Gegenstandes das Schmutzwasser mit den Keimen von dessen Oberfläche aufsaugt und eine saubere und keimarme Oberfläche hinterläßt.

Beim Reinigen steriler Bereiche in Krankenhäusern, Labors und Arztpraxen besteht gewissermaßen ein Zielkonflikt zwischen möglichst saugfähigen Reinigungsmaterialien einerseits, um Schmutzwasser und Keime aufzunehmen und Reinigungsmaterialien mit einer möglichst geringen Saugfähigkeit andererseits, um zu verhindern, daß sich Keime im Reinigungsmaterial ablagern.

Es ist Aufgabe der vorliegenden Erfindung, eine Reinigungsvorrichtung zum Reinigen von flächigen Gegenständen der eingangs genannten Art dahingehend weiterzubilden, daß einerseits ein sicherer Halt des Reinigungskissens an dem Hohlkörper gewährleistet ist, und anderseits verhindert wird, daß Keime innerhalb steriler Bereiche in Krankenhäusern, Labors und Arztpraxen verschleppt werden.

Zur Lösung dieser Aufgabe schlägt die Erfindung ausgehend von einer Reinigungsvorrichtung der eingangs genannten Art vor, daß das Reinigungskissen mittels Klettverbindungselementen lösbar an dem Hohlkörper festgelegt ist.

Bei der Reinigungsvorrichtung gemäß der Erfindung ist das Reinigungskissen durch die Klettverbindungselemente so an dem Hohlkörper festgelegt, daß es sich auch unter dem Einfluß von verformenden Scherkräften nicht von dem Hohlkörper ablöst. Durch die hohe Saufähigkeit des Reinigungskissens werden Schmutzwasser und Keimen sicher aufgesaugt und die zu reinigenden, flächigen Gegenstände sauber und keimarm. Nach dem Reinigen eines Raumes oder Bereiches in Krankenhäusern, Labors oder Arztpraxen ist das Reinigungskissen mit Schmutzwasser und Keimen vollgesogen. Die Keime sind in Poren des Reinigungskissens gespeichert. Auf dem Hohlkörper der Reinigungsvorrichtung, der aus nicht saugfähigem Material besteht, befinden sich demgegenüber nur wenige Keime.

Bei der erfindungsgemäßen Reinigungsvorrichtung wird nun nicht nur die äußerste, schmutzige Schicht des Reinigungsmaterials erneuert, sondern es werden die gesamten saugfähigen Teile der Reinigungsvorrichtung von den nicht saugfähigen getrennt, d.h., es werden gewissermaßen die mit Keimen belasteten Teile von den keimfreien Teilen getrennt. Die benutzten, abgelösten Reinigungskissen werden entweder gereinigt, desinfiziert und wiederverwendet oder aber direkt dem Abfall zugeführt. Der Hohlkörper selbst kann ggf. auf einfache Weise von Keimen befreit werden, beispielsweise durch das Eintauchen in eine Desinfektionslösung.

In einer bevorzugten Ausführung der Erfindung wird vorgeschlagen, daß das Reinigungskissen Oberflächen aus stabilem, widerstandsfähigem Textilgewebe aus Natur- oder Kunststoffasern aufweist. Solche Textil-Reinigungskissen aus stabilem Material haben eine besonders gute Reinigungswirkung und können besonders häufig wiederverwendet werden.

Die Wiederverwendung der Textil-Reinigungskissen reduziert das Abfallaufkommen in Krankenhäusern, Labors oder Arztpraxen erheblich. Durch häufiges Wechseln der Textil-Reinigungskissen während des Reinigungsvorganges kann äußerste Keimfreiheit garantiert werden, ohne daß dies zu einer erhöhten Abfallmenge führt.

In einer vorteilhaften Weiterbildung der Erfindung wird vorgeschlagen, daß das Reinigungskissen lediglich auf den Reinigungsflächen des Hohlkörpers angeordnet ist. Derartig angeordnete Reinigungskissen lassen sich besonders leicht auf dem Hohlkörper anbringen und von diesem wieder abtrennen. Der Benutzer der Reinigungsvorrichtung kann diese in den Bereichen halten, die nicht von dem Reinigungskissen bedeckt sind, ohne feuchte oder schmutzige Hände zu bekommen.

Die Reinigungsvorrichtung läßt sich besonders vielseitig einsetzen, wenn die Reinigungskissen unterschiedliche Oberflächenmaterialien aufweisen. Diese Oberflächenmaterialien sind sinnvollerweise auf die zu reinigende Fläche und die Art des zu beseitigenden Schmutzes abgestimmt.

Als besonders vorteilhaft hat sich im praktischen Einsatz erwiesen, den Hohlkörper quaderförmig auszubilden und auf einer von zwei sich gegenüberliegenden Reinigungsflächen ein Reinigungskissen mit einer Oberfläche aus einem rauhen Scheuergewebe und auf der anderen ein Reinigungskissen mit einer Oberfläche aus einem weichen Wischgewebe lösbar zu befestigen. Angetrocknete Schmutzränder lassen sich besonders einfach mit dem rauhen Scheuergewebe entfernen. Die aufgeweichten und von der zu reinigenden Fläche abgelösten Schmutzteilchen können dann mit dem weichen Wischgewebe aufgenommen werden.

In einer weiteren vorteilhaften Ausgestaltungsform der Erfindung wird vorgeschlagen, daß das Reinigungskissen als Manschette ausgebildet ist und um den gesamten Hohlkörper verläuft. Die Manschette wird im ganzen über den Hohlkörper gezogen und auf diesem mittels Klettverbindungselementen gegen ein Verrutschen gesichert. Ein Reinigungskissen in Form einer Manschette bietet insbesondere dann Vorteile, wenn auf äußerste Keimfreiheit Wert gelegt wird. Durch das Anordnen einer frischen, keimfreien Manschette um den Hohlkörper ist dieser ganz sicher zu allen Seiten keim-frei. Zu reinigende Gegenstände lassen sich mittels der Manschette auch im Bereich von Nischen und Ecken problemlos und effektiv reinigen.

Die Manschette läßt sich besonders leicht an dem Hohlkörper befestigen und von diesem wieder abtrennen, wenn sie einen Klettverschluß aufweist. Dieser verläuft vorteilhafterweise in Längsrichtung des Hohlkörpers und erlaubt es, die Manschette zu öffnen, sie um den Hohlkörper zu legen und durch Schließen des Klettverschlusses diese an ihm zu befestigen. Auch hierbei ist es sinnvoll, wenn die Manschette mittels eines Klettverbindungselements an dem Hohlkörper befestigt ist und ein Verrutschen der Manschette in Längsrichtung des Hohlkörpers unterbunden wird.

Die Reinigungsvorrichtung mit der Manschette läßt sich besonders vielseitig einsetzen, wenn die Manschette im Bereich der Reinigungsflächen unterschiedliche Oberflächenmaterialien aufweist.

In einer besonders vorteilhaften Weiterbildung der erfindungsgemäßen Reinigungsvorrichtung wird vorgeschlagen, daß ein Klettverbindungselement auf einer Reinigungsfläche des Hohlkörpers geklebt ist. Dieses Klettverbindungselement weist viele kleine Häkchen auf und greift in ein korrespondierendes Klettverbindungselement mit einer Vielzahl kleiner Ösen auf der Rückseite des Reinigungsmaterials bzw. der Innenseite der Manschette. Diese Klettverbindungselemente mit Ösen werden auf der Rückseite des Reinigungskissens bzw der Innenseite der Manschette beispielsweise genäht oder sind ein integraler Bestandteil des Reinigungsmaterials selbst. Selbstverständlich können die Klettverbindungselemente auch umgekehrt angeordnet sein, d.h., daß an dem Hohlkörper das Klettverbindungselement mit den ösen und and em Reinigungsmaterial das mit den Häkchen angeordnet sind.

Eine weitere vorteilhafte Ausführungsform der Erfindung sieht vor, daß ein Klettverbindungselement integraler Bestandteil einer Reinigungsfläche des Hohlkörpers ist. Dies hat den Vorteil, daß der Hohlkörper mit Klettverbindungselement in einem Arbeitsgang hergestellt werden kann.

Für den Einsatz der erfindungsgemäßen Reinigungsvorrichtung in sterilen Bereichen in Krankenhäusern, Laboren und Arztpraxen ergeben sich besondere Vorteile, wenn Auslaß- und Einfüllöffnung als abschraubbarer Sprühverschluß ausgebildet sind. Dies ermöglicht ein Auftragen von Reinigungs- oder Desinfektionsmittel auf den zu reinigenden Gegenstand, ohne diesen jedoch zu berühren und ohne Keime auf ihn zu übertragen. Durch den Sprühverschluß wird das Reinigungs-bzw. Desinfektionsmittel fein und gleichmäßig auf den zu reinigenden Gegenstand verteilt. Der Sprühverschluß kann einfach mit dem Zeigefinger der Hand des Benutzers, die die Reinigungsvorrichtung hält, bedient werden. Durch Abschrauben des Sprühverschlusses wird eine Einfüllöffnung zugänglich, durch die das Reinigungs- oder Desinfektionsmittel in den Hohlkörper der Reinigungsvorrichtung gefüllt werden kann.

Um die Herstellungskosten der erfindungsgemäßen Vorrichtung zu reduzieren, ist es vorteilhaft, wenn Auslaß- und Einfüllöffnung getrennt ausgebildet sind. Dabei kann das Reinigungs- oder Desinfektionsmittel über hinter dem Reinigungsmaterial angeordnete Auslaßöffnung auf die zu reinigenden flächigen Gegenstände gelangen. Die Auslaßöffnungen sind als einfache kleine Öffnungen ausgebildet, mit denen der Hohlkörper problemlos zu versehen ist. Die Einfüllöffnung kann ebenfalls als einfache Öffnung ausgebildet sein, die beispielsweise durch einen Klemmverschluß verschlossen ist.

Im folgenden werden an Hand der Zeichnungen mehrere Ausführungsbeispiele der Erfindung näher erläutert.

Es zeigen:
- Fig. 1: eine erfindungsgemäße Reinigungsvorrichtung in einer ersten Ausführungsform;
- Fig. 2: eine erfindungsgemäße Reinigungsvorrichtung in einer zweiten Ausführungsform.

In den folgenden Zeichnungen sind die einzelnen Bauteile mit übereinstimmenden Bezugszeichen versehen.

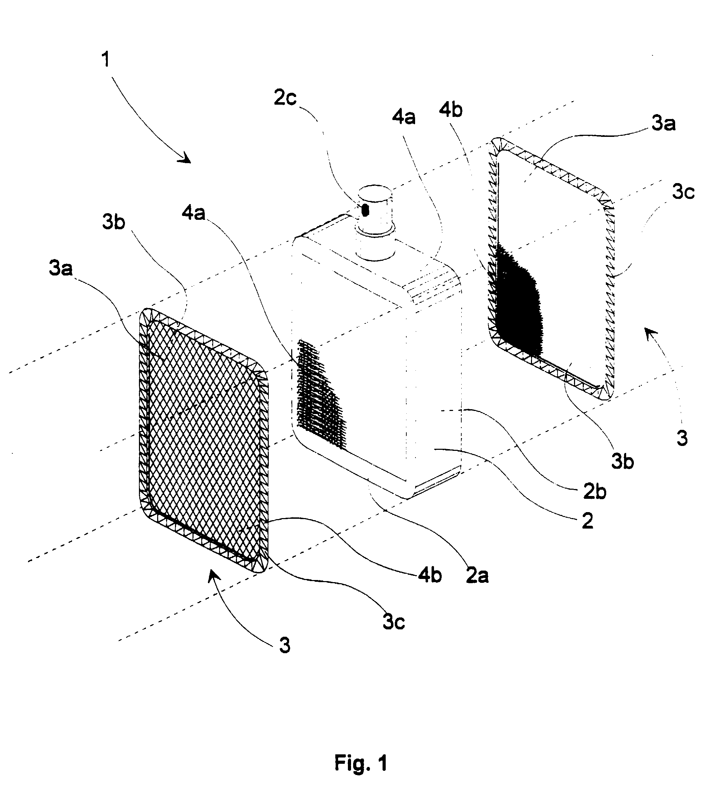

In Fig. 1 ist eine erfindungsgemäße Reinigungsvorrichtung in ihrer Gesamtheit mit dem Bezugszeichen 1 gekennzeichnet. Sie weist einen Hohlkörper 2 auf, der mit Reinigungs- oder Desinfektionsmittel gefüllt ist. Der Hohlkörper 2 verfügt über zwei ebene sich gegenüberliegend angeordnete Reinigungsflächen 2a, 2b. Um das Reinigungs- oder Desinfektioinsmittel aus dem Hohlkörper 2 auf einen zu reinigenden flächigen Gegenstand aufzutragen, ist oben auf dem Hohlkörper 2 ein abschraubbarer Sprühverschluß 2c angeordnet. Nach dem Abschrauben des Sprühverschlusses 2c gibt dieser eine große Einfüllöfnung frei, durch die der Hohlkörper 2 mit Reinigungs- oder Desinfektionsmittel auffüllbar ist.

An diesen Reinigungsflächen 2a, 2b werden Reinigungskissen 3 lösbar befestigt. Die Reinigungskissen 3 weisen Oberflächen 3a aus einem stabilen, widerstandsfähigen Textilgewebe aus Natur- oder Kunststoffasern auf. Die Oberflächen 3a der Reinigungskissen 3 bestehen beispielsweise aus einem rauhen Scheuergewebe oder einem weichen Wischgewebe. Die Reinigungskissen 3 werden mittels Klettverbindungselementen 4a, 4b an den ebenen Reinigungsflächen 2a, 2b des Hohlkörpers 2 lösbar befestigt. Dabei greifen kleine Ösen eines auf der Rückseite 3b der Reinigungskissen 3 angeordneten Klettverbindungselementes 4b in ein korrespondierendes auf der ebenen Reinigungsfläche 2a, 2b des Hohlkörpers 2 angeordnetes mit Häkchen versehenes Klettverbindungselement 4a. Die Klettverbindungselemente 4a mit Häkchen sind auf die Reinigungsflächen 2a des Hohlkörpers 2 mittels Klebstoff befestigt. Die Klettverbindungselemente 4b mit kleinen Ösen sind integraler Bestandteil eines rückwärtigen Textilgewebes der Reinigungskissen 3. Das rückwärtige Textilgewebe der Rückseite 3b und das Oberflächenmaterial der Oberfläche 3a des Reinigungskissens 3 sind mittels einer an ihrem Rand verlaufenden Naht 3c miteinander verbunden.

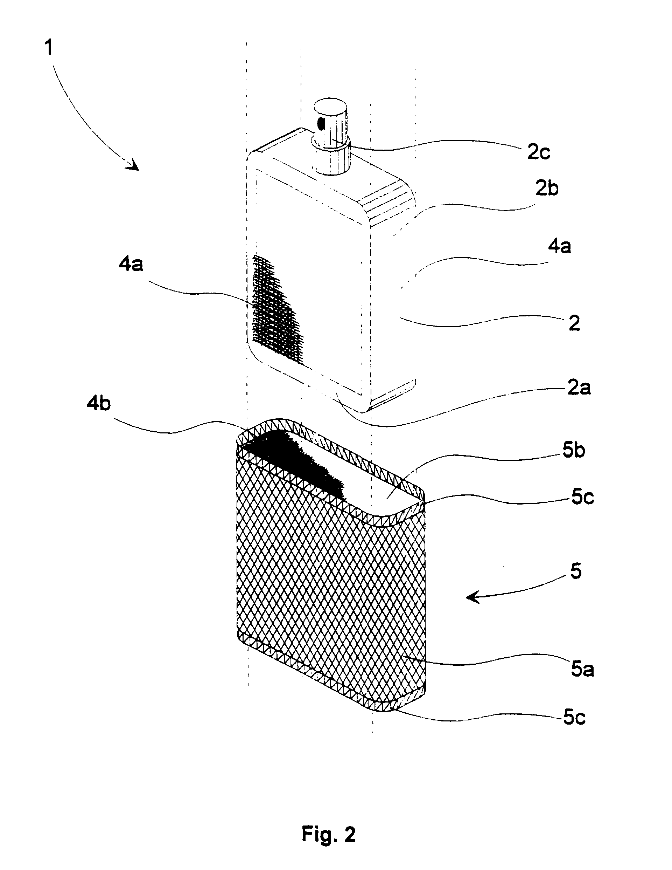

In der zweiten Ausführungsform der erfindungsgemäßen Reinigungsvorrichtung 1 aus Fig. 2 ist das Reinigungskissen 3 als eine Manschette 5 ausgebildet. Sie besteht aus einem äußeren Oberflächenmaterial 5a aus einem rauhen Scheuergewebe oder einem weichen Wischgewebe und einem inneren Textilgewebe 5b, in welches kleine Ösen des Klettverbindungselementes 4b integriert sind. Das innere Textilgewebe 5b und das äußere Oberflächenmaterial 5a sind mittels einer an ihrem Rand verlaufenden Naht 5c miteinander verbunden. Die Manschette 5 wird von oben oder von unten über den Hohlkörper 2 gezogen und an ihm mittels Klettverbindungselementen 4a, 4b gegen ein Verrutschen in Längsrichtung gesichert.

## Patentansprüche

1. Reinigungsvorrichtung (1) zum Reinigen von flächigen Gegenständen, insbesondere steriler Bereiche in Krankenhäusern, Labors und Arztpraxen, wobei die Reinigungsvorrichtung einen mit mindestens einer ebenen Reinigungsfläche (2a, 2b) und mindestens einer Auslaß- und Einfüllöffnung versehenen und mit Reinigungs- oder Desinfektionsmittel gefüllten Hohlkörper (2) aufweist, an dem saugfähiges Reinigungsmaterial in Form eines Reinigungskissens (3) befestigt ist, welches in einem Stück von dem Hohlkörper (2) abtrennbar ist, **dadurch gekennzeichnet,** daß das Reinigungskissen (3) mittels Klettverbindungselementen (4a, 4b) lösbar an dem Hohlkörper (2) festgelegt ist.

2. Reinigungsvorrichtung (1) nach Anspruch 1, dadurch gekennzeichnet, daß das Reinigungskissen (3) Oberflächen aus stabilem, widerstandsfähigem Textilgewebe aus Natur- oder Kunststoffasern aufweist.

3. Reinigungsvorrichtung (1) nach Anspruch 1, dadurch gekennzeichnet, daß Reinigungskissen (3) lediglich auf den Reinigungsflächen (2a, 2b) des Hohlkörpers (2) angeordnet sind.

4. Reinigungsvorrichtung (1) nach Anspruch 3, dadurch gekennzeichnet, daß die Reinigungskissen (3) unterschiedliche Oberflächenmaterialien aufweisen.

5. Reinigungsvorrichtung (1) nach Anspruch 4, dadurch gekennzeichnet, daß der Hohlkörper (2) quaderförmig ausgebildet ist und auf einer von zwei sich gegenüberliegenden Reinigungsflächen (2a, 2b) ein Reinigungskissen (3) mit einer Oberfläche aus einem rauhen Scheuergewebe und auf der anderen ein Reinigungskissen (3) mit einer Oberfläche aus einem weichen Wischgewebe lösbar befestigt sind.

6. Reinigungsvorrichtung (1) nach Anspruch 1, dadurch gekennzeichnet, daß das Reinigungskissen (3) als Manschette (5) ausgebildet ist, die um den gesamten Hohlkörper (2) verläuft und einen Klettverschluß aufweist.

7. Reinigungsvorrichtung (1) nach Anspruch 6, dadurch gekennzeichnet, daß die Manschette (5) im Bereich der Reinigungsflächen (2a, 2b) unterschiedliche Oberflächenmaterialien aufweist.

8. Reinigungsvorrichtung (1) nach Anspruch 1, dadurch gekennzeichnet, daß ein Klettverbindungselement (4a) auf einer Reinigungsfläche (2a, 2b) des Hohlkörpers (3) geklebt ist.

9. Reinigungsvorrichtung (1) nach Anspruch 1, dadurch gekennzeichnet, daß ein Klettverbindungselement (4a) integraler Bestandteil einer Reinigungsfläche (2a, 2b) des Hohlkörpers (2) ist.

10. Reinigungsvorrichtung (1) nach einem oder mehreren der Ansprüche 1 bis 9, dadurch gekennzeichnet, daß Auslaß- und Einfüllöffnung des Hohlkörpers (2) als abschraubbarer Sprühverschluß (2c) ausgebildet sind.

11. Reinigungsvorrichtung (1) nach einem oder mehreren der Ansprüche 1 bis 9, dadurch gekennzeichnet, daß Auslaß- und Einfüllöffnung des Hohlkörpers (2) getrennt ausgebildet sind.

12. Reinigungsvorrichtung (1) nach Anspruch 11, dadurch gekennzeichnet, daß das Reinigungs- oder Desinfektionsmittel über hinter dem Reinigungskissen (3) angeordnete Auslaßöffnungen auf die zu reinigenden flächigen Gegenstände gelangt.

## Claims

1. A cleaning device (1) for cleaning flat articles. in particular sterile areas in hospitals. laboratories and medical practices. wherein the cleaning device has a hollow body (2) which is provided with at least one cleaning surface (2a. 2b) and at least one outlet and filling opening and which is filled with cleaning or disinfecting agent and to which absorbent cleaning material is fixed in the form of a cleaning pad (3) which is separable in one piece from the hollow body (2). characterised in that the cleaning pad (3) is releasably secured to the hollow body (2) by means of touch-and-close connecting elements (4a. 4b).

2. A cleaning device (1) according to claim 1 characterised in that the cleaning pad (3) has surfaces of stable. resistant textile cloth of natural or synthetic fibres.

3. A cleaning device (1) according to claim 1 characterised in that cleaning pads (3) are arranged only on the cleaning surfaces (2a. 2b) of the hollow body (2).

4. A cleaning device (1) according to claim 3 characterised in that the cleaning pads (3) have different surface materials.

5. A cleaning device (1) according to claim 4 characterised in that the hollow body (2) is of a cuboidal configuration and a cleaning pad (3) with a surface comprising a rough scouring material is releasably fixed on one of two mutually oppositely disposed cleaning surfaces (2a. 2b) and a cleaning pad (3) with a surface comprising a soft wiping material is releasably fixed on the other.

6. A cleaning device (1) according to claim 1 characterised in that the cleaning pad (3) is in the form of a sleeve (5) which extends around the entire hollow body (2) and has a touch-and-close fastener.

7. A cleaning device (1) according to claim 6 characterised in that the sleeve (5) has different surface materials in the region of the cleaning surfaces (2a. 2b).

8. A cleaning device (1) according to claim 1 characterised in that a touch-and-close connecting element (4a) is glued on a cleaning surface (2a. 2b) of the hollow body (3).

9. A cleaning device (1) according to claim 1 characterised in that a touch-and-close connecting element (4a) is an integral component of a cleaning surface (2a, 2b) of the hollow body (2).

10. A cleaning device (1) according to one or more of claims 1 to 9 characterised in that the outlet and filling opening of the hollow body (2) are in the form of a spray closure (2c) which can be unscrewed.

11. A cleaning device (1) according to one or more of claims 1 to 9 characterised in that the outlet and filling opening of the hollow body (2) are provided separately.

12. A cleaning device (1) according to claim 11 characterised in that the cleaning or disinfecting agent arrives at the flat articles to be cleaned by way of outlet openings arranged behind the cleaning pad (3).

## Revendications

1. Dispositif de nettoyage (1) pour nettoyer des objets plans, notamment des zones stériles dans des hôpitaux, laboratoires et cabinets médicaux, où le dispositif de nettoyage présente un corps creux (2) pourvu d'au moins une face de nettoyage plane (2a, 2b) et d'au moins une ouverture de sortie et de remplissage et est rempli de produits de nettoyage ou de désinfection, auquel est fixé un matériau de nettoyage aspirant sous la forme d'un coussin de nettoyage (3), qui est séparable en une pièce du corps creux (2), caractérisé en ce que le coussin de nettoyage (3) est fixé par des éléments auto-accorchants (4a, 4b) amoviblement au corps creux (2).

2. Dispositif de nettoyage (1) selon la revendication 1, caractérisé en ce que le coussin de nettoyage (3) présente des surfaces en un tissu textile stable et résistant en fibres naturelles ou en fibres de matière synthétique.

3. Dispositif de nettoyage (1) selon la revendication 1, caractérisé en ce que les coussins de nettoyage (3) sont disposés uniquement sur les faces de nettoyage (2a, 2b) du corps creux (2).

4. Dispositif de nettoyage (1) selon la revendication 3, caractérisé en ce que les coussins de nettoyage (3) présentent des matériaux de surface différents.

5. Dispositif de nettoyage (1) selon la revendication 4, caractérisé en ce que le corps creux (2) a une forme parallélépipède et que sont fixées amoviblement sur une de deux faces de nettoyage opposées (2a, 2b) un coussin de nettoyage (3) avec une surface en un tissu de frottement rugueux et sur l'autre un coussin de nettoyage (3) avec une surface en un tissu d'essuyage mou.

6. Dispositif de nettoyage (1) selon la revendication 1, caractérisé en ce que le coussin de nettoyage (3) est réalisé comme manchette (5) qui s'étend autour de tout le corps creux (2) et qui présente une fermeture auto-accrochante.

7. Dispositif de nettoyage (1) selon la revendication 6, caractérisé en ce que la manchette (5) présente au voisinage des faces de nettoyage (2a, 2b) des matériaux de surface différents.

8. Dispositif de nettoyage (1) selon la revendication 1, caractérisé en ce qu'un élément auto-accrochant (4a) est collé sur une face de nettoyage (2a, 2b) du corps creux (3).

9. Dispositif de nettoyage (1) selon la revendication 1, caractérisé en ce qu'un élément auto-accrochant (4a) est un composant intégral d'une face de nettoyage (2a, 2b) du corps creux (2).

10. Dispositif de nettoyage (1) selon l'une ou plusieurs des revendications 1 à 9, caractérisé en ce que les ouvertures de sortie et de remplissage du corps creux (2) sont réalisées comme bouchon pulvérisateur (2c) pouvant être dévissé.

11. Dispositif de nettoyage (1) selon l'une ou plusieurs des revendications 1 à 9, caractérisé en ce que les ouvertures de sortie et de remplissage du corps creux (2) sont réalisées séparément.

12. Dispositif de nettoyage (1) selon la revendication 11, caractérisé en ce que le produit de nettoyage ou de désinfection arrive par des ouvertures de sortie disposées derrière le coussin de nettoyage (3) sur les objets plans à nettoyer.
